# EUROPEAN PATENT SPECIFICATION

(11) **EP 4 467 007 B1**
(45) Date of publication and mention of the grant of the patent: **04.11.2026**
(21) Application number: 23835112.6
(22) Date of filing: 31.03.2023
(51) Int. Cl.: A61K 36/185, A23L 33/105, A23F 3/34, A23F 3/36, A23L 2/38, A23L 2/80, A61K 8/9789, A61P 3/04, A61Q 19/00

(54) **CAFFEINE-REMOVED ILEX PARAGUARIENSIS EXTRACT, ANTI-OBESITY COMPOSITION, METHOD FOR REMOVING CAFFEINE, AND METHOD FOR PRODUCING ILEX PARAGUARIENSIS EXTRACT**
EXTRAKT AUS ILEX PARAGUARIENSIS OHNE KOFFEIN, ZUSAMMENSETZUNG GEGEN ADIPOSITAS, VERFAHREN ZUR ENTFERNUNG VON KOFFEIN UND VERFAHREN ZUR HERSTELLUNG DES ILEX PARAGUARIENSIS-EXTRAKTS
EXTRAIT D'ILEX PARAGUARIENSIS DONT LA CAFÉINE A ÉTÉ RETIRÉE, COMPOSITION ANTI-OBÉSITÉ, PROCÉDÉ DE RETRAIT DE CAFÉINE ET PROCÉDÉ DE PRODUCTION D'EXTRAIT D'ILEX PARAGUARIENSIS

(30) Priority: 08.07.2022 JP 2022110805
(43) Date of publication of application: 27.11.2024
(73) Proprietor: Tokiwa Phytochemical Co., Ltd., Sakura-shi, Chiba 285-0801 (JP)
(72) Inventor: YANG, Jinwei, Sakura-shi, Chiba 285-0801 (JP); UCHIYAMA, Shinji, Sakura-shi, Chiba 285-0801 (JP); KOBAYASHI, Yukiko, Sakura-shi, Chiba 285-0801 (JP)
(74) Representative: Manitz Finsterwald Patent- und Rechtsanwaltspartnerschaft mbB
(86) International application number: PCT/JP2023/013504
(87) International publication number: WO 2024/009571

(56) References cited:
- WO-A1-2005/067952
- WO-A1-2011/105042
- WO-A1-2015/138556
- WO-A1-98/42209
- JP-A- 2001 518 920
- JP-A- 2007 517 830
- JP-A- 2008 088 187
- JP-A- 2010 241 800
- JP-A- 2014 140 348
- JP-A- 2014 140 348
- JP-A- 2014 212 743
- JP-A- 2019 126 274
- JP-A- H0 870 772
- JP-A- H06 142 405
- JP-B2- 6 734 075
- US-A1- 2016 081 967
- US-B2- 10 426 178
- US-B2- 7 279 184
- BALZAN SILVIE ET AL: "Lipid-lowering effects of standardized extracts ofIlex paraguariensisin high-fat-diet rats", FITOTERAPIA, IDB HOLDING, MILAN, IT, vol. 86, 17 February 2013 (2013-02-17), pages 115 - 122, XP028579855, ISSN: 0367-326X, DOI: 10.1016/J.FITOTE.2013.02.008
- SANTO ALEXANDRE TIMM DO ESPIRITO ET AL: "Decaffeination of yerba mate by supercritical fluid extraction: Improvement, mathematical modelling and infusion analysis", THE JOURNAL OF SUPERCRITICAL FLUIDS, ELSEVIER, AMSTERDAM, NL, vol. 168, 22 October 2020 (2020-10-22), XP086357859, ISSN: 0896-8446, [retrieved on 20201022], DOI: 10.1016/J.SUPFLU.2020.105096
- CASSEL E ET AL: "Supercritical fluid extraction of alkaloids from Ilex paraguariensis St. Hil", JOURNAL OF FOOD ENGINEERING, ELSEVIER, AMSTERDAM, NL, vol. 100, no. 4, 1 October 2010 (2010-10-01), pages 656 - 661, XP027119680, ISSN: 0260-8774, [retrieved on 20100524]

## Description

### [Field of invention]

This invention relates to a decaffeinated yerba mate extract, an anti-obesity composition, a decaffeination method and a method for the production of a yerba mate extract.

### [Background of invention]

Yerba mate (Ilex paraguariensis) is an evergreen tree belonging to the genus Ilex in the Aquifoliaceae family and is native to Argentina, Paraguay, Brazil and other South American countries. Yerba mate, which is rich in vitamins and minerals, is also known as "drinking salad," and yerba mate tea, which is brewed by infusing yerba mate leaves with hot or cold water, has been consumed in South America as an important source of nutrition since ancient times.

It has been reported that yerba mate has physiological activities such as anti-obesity, anticholesterol, anti-diabetic and fat-burning promoting effects (see, for example, Non-patent documents 1 and 2). Caffeine and polyphenols are known as the active ingredients of yerba mate. Regarding caffeine, Non-patent document 3 reports that caffeine from yerba mate extracted using the supercritical carbon dioxide extraction method showed an inhibitory effect on fat accumulation in cell and animal tests, whereas leaf extract after caffeine extraction showed no effect. It also reports that caffeine from yerba mate reduced gene expression of fatty acid synthase, whereas leaf extracts after caffeine extraction showed no change, suggesting that caffeine in yerba mate has an anti-obesity effect due to its inhibition of fat synthesis.

Meanwhile, caffeine is known to excite nerves by inhibiting adenosine receptors and has awakening, diuretic and gastric acid secretion-promoting effects. Consuming it late at night can lead to poor sleep, and taking it on an empty stomach can upset the stomach. Overdose causes dizziness, increased heart rate, agitation, anxiety, tremors, insomnia, diarrhea, nausea and other symptoms. Longterm consumption may increase the risk of hypertension, and high concentrations of caffeine in pregnant women may lead to low birth weight. These negative effects of caffeine have increased demand for decaffeinated and non-caffeinated food and beverages, with the market for decaf beverages packed in plastic bottles expected to reach approximately 13 billion yen in FY2025.

Yerba mate also contains polyphenols, caffeoylquinic acids and dicaffeoylquinic acids, which are ester compounds of caffeic acid and quinic acid. Non-patent document 4 reports that administration of chlorogenic acid (one of the caffeoylquinic acids) and 3,5-dicaffeoylquinic acid isolated from a hot water extract of yerba mate significantly reduces food intake in mice. It has been reported that caffeoylquinic acids and dicaffeoylquinic acids have pancreatic lipase inhibitory effects (see, for example, Non-patent document 5), suggesting that these components may also have anti-obesity and metabolism-improving effects.
[Non-patent document 1] Hussein, G. M., Matsuda, H., Nakamura, S., Akiyama, T., Tamura, K., & Yoshikawa, M. (2011). Protective and ameliorative effects of mate (Ilex paraguariensis) on metabolic syndrome in TSOD mice. Phytomedicine, 19(1), 88-97.
[Non-patent document 2] Dos Santos, T. W., Miranda, J., Teixeira, L., Aiastui, A., Matheu, A., Gambero, A., Portillo, M. P., & Ribeiro, M. L. (2018). Yerba Mate Stimulates Mitochondrial Biogenesis and Thermogenesis in High-Fat-Diet-Induced Obese Mice. Molecular Nutrition & Food Research, 62(15), 1800142.
[Non-patent document 3] Zapata, F. J., Rebollo-Hernanz, M., Novakofski, J. E., Nakamura, M. T., & de Mejia, E. G. (2020). Caffeine, but not other phytochemicals, in mate tea (Ilex paraguariensis St. Hilaire) attenuates high-fat-high-sucrose-diet-driven lipogenesis and body fat accumulation. Journal of Functional Foods, 64, 103646.
[Non-patent document 4] Hussein, G. M. E., Matsuda, H., Nakamura, S., Hamao, M., Akiyama, T., Tamura, K., & Yoshikawa, M. (2011). Mate tea (Ilex paraguariensis) promotes satiety and body weight lowering in mice: involvement of glucagon-like peptide-1. Biological and Pharmaceutical Bulletin, 34(12), 1849-1855.
[Non-patent document 5] Narita, Y., Iwai, K., Fukunaga, T., & Nakagiri, O. (2012). Inhibitory activity of chlorogenic acids in decaffeinated green coffee beans against porcine pancreas lipase and effect of a decaffeinated green coffee bean extract on an emulsion of olive oil. Bioscience, Biotechnology, and Biochemistry, 76(12), 2329-2331.
[Non-patent document 6] Arçari, D. P., Bartchewsky, Jr., W., Dos Santos, T. W., Oliveira, K. A., DeOliveira, C. C., Gotardo, É. M., Pedrazzoli, Jr., J., Gambero, A., Ferraz, L. F. C., Carvalho, P. O., & Ribeiro, M. L. (2011). Anti-inflammatory effects of yerba maté extract (Ilex paraguariensis) ameliorate insulin resistance in mice with high fat diet-induced obesity. Molecular and Cellular Endocrinology, 335(2), 110-115.
[Non-patent document 7] Conceição, E. P. S., Kaezer, A. R., Peixoto-Silva, N., Felzenszwalb, I., De Oliveira, E., Moura, E. G., & Lisboa, P. C. (2017). Effects of Ilex paraguariensis (yerba mate) on the hypothalamic signalling of insulin and leptin and liver dysfunction in adult rats overfed during lactation. Journal of Developmental Origins of Health and Disease, 8(1), 123-132.
[Non-patent document 8] Machado, M. L., Arantes, L. P., Gubert, P., Zamberlan, D. C., da Silva, T. C., da Silveira, T. L., Boligon, A., & Soares, F. A. A. (2018). Ilex paraguariensis modulates fat metabolism in Caenorhabditis elegans through purinergic system (ADOR-1) and nuclear hormone receptor (NHR-49) pathways. PLOS ONE, 13(9), e0204023.
[Non-patent document 9] Balzan, S., Hernandes, A., Reichert, C. L., Donaduzzi, C., Pires, V. A., Gasparotto, Jr., A., & Cardozo, Jr., E. L. (2013). Lipid-lowering effects of standardized extracts of Ilex paraguariensis in high-fat-diet rats. Fitoterapia, 86, 115-122.
[Non-patent document 10] Gao, H., Liu, Z., Qu, X., & Zhao, Y. (2013). Effects of Yerba Mate tea (Ilex paraguariensis) on vascular endothelial function and liver lipoprotein receptor gene expression in hyperlipidemic rats. Fitoterapia, 84, 264-272.
[Non-patent document 11] Carmo, L. S., Rogero, M. M., Cortez, M., Yamada, M., Jacob, P. S., Bastos, D. H. M., Borelli, P., & Fock, R. A. (2013). The effects of yerba maté (Ilex paraguariensis) consumption on IL-1, IL-6, TNF-α and IL-10 production by bone marrow cells in wistar rats fed a high-fat diet. International Journal for Vitamin and Nutrition Research, 83(1), 26-35.

### [Summary of invention]

### [Problems to be solved by invention]

The documents reporting anti-obesity and metabolism-improving effects show the content of caffeine, caffeoylquinic acids and dicaffeoylquinic acids in a yerba mate extract as in Table 1. Caffeine content and the total content of caffeoylquinic acids and dicaffeoylquinic acids range from 5.82 to 35.4 mg/g and from 3.7 to 180.5 mg/g, respectively. Only Non-patent document 9 shows a caffeine content of 10 mg/g (1.0% by weight) or less and a total content of caffeoylquinic acids and dicaffeoylquinic acids of 100 mg/g (10% by weight) or more. However, the yerba mate extract in Non-patent document 9 was produced by fractionating a 70% ethanol extract of yerba mate with n-butanol, which cannot be used for food applications.

**[Table 1]**

| Document | Caffeine | Caffeoylquinic acids and dicaffeoylquinic acids |
|---|---|---|
| Non-patent document 1 | 35.4 mg/g | 38.5 mg/g |
| Non-patent document 6 | 5.82 mg/g | 32.25 mg/g |
| Non-patent document 7 | 21.00 mg/L | 41.20 mg/L |
| Non-patent document 8 | 8.8 mg/g | 3.7 mg/g |
| Non-patent document 9 | 6.52 mg/g | 180.51 mg/g |
| Non-patent document 10 | 12.3 mg/g | 21.1 mg/g |
| Non-patent document 11 | 14.53 mg/g | 27.26 mg/g |

The present invention was made in view of the above problems to provide a method for the production of a safer yerba mate (Ilex paraguariensis) extract prepared by removing caffeine to 1.0% or less by weight while containing 10% or more by weight in total of caffeoylquinic acids and dicaffeoylquinic acids, which can be used in foods, and a composition obtained therefrom.

### [Means to solve problems]

As a result of the inventor's diligent examination of methods for the production of a yerba mate (Ilex paraguariensis) with respect to the above problems, a method for the production of a yerba mate extract with caffeine removed to 1.0% or less by weight with an adsorbent has been successfully developed. In addition, the total content of caffeoylquinic acids and dicaffeoylquinic acids was maintained at 10% or more by weight, and the inhibitory effect on fat accumulation was demonstrated in cell tests, leading to the completion of the present invention.

A method for the production of a decaffeinated yerba mate extract according to one aspect of the present invention comprises a step of removing caffeine from a yerba mate (Ilex paraguariensis) crude extract with an adsorbent.

A yerba mate extract according to another aspect of the present invention is prepared by removing caffeine from a yerba mate (Ilex paraguariensis) crude extract with an adsorbent and comprises 1.0% or less by weight of caffeine and 10% or more by weight in total of caffeoylquinic acids and dicaffeoylquinic acids.

A yerba mate extract according to another aspect of the present invention is prepared by removing caffeine from a yerba mate (Ilex paraguariensis) crude extract with an adsorbent and comprises 0.5% or less by weight of caffeine and 15% or more by weight in total of caffeoylquinic acids and dicaffeoylquinic acids.

An anti-obesity composition according to another aspect of the present invention comprises as an active ingredient a yerba mate extract prepared by removing caffeine from a yerba mate (Ilex paraguariensis) crude extract with an adsorbent and comprising 1.0% or less by weight of caffeine and 10% or more by weight in total of caffeoylquinic acids and dicaffeoylquinic acids.

An anti-obesity composition according to another aspect of the present invention comprises as an active ingredient a yerba mate extract prepared by removing caffeine from a yerba mate (Ilex paraguariensis) crude extract with an adsorbent and comprising 0.5% or less by weight of caffeine and 15% or more by weight in total of caffeoylquinic acids and dicaffeoylquinic acids.

An anti-obesity food composition, an anti-obesity pharmaceutical composition or an anti-obesity cosmetic composition according to another aspect of the present invention comprises as an active ingredient a yerba mate extract according to any of the above aspects, the yerba mate extract prepared by removing caffeine from a yerba mate (Ilex paraguariensis) crude extract with an adsorbent.

A decaffeination method according to another aspect of the present invention comprises a step of removing caffeine from a yerba mate (Ilex paraguariensis) crude extract with an adsorbent.

### [Effects of invention]

As described above, the present invention provides a decaffeinated yerba mate extract, a method for the production thereof and an anti-obesity composition comprising a yerba mate extract as an active ingredient.

### [Brief description of drawings]

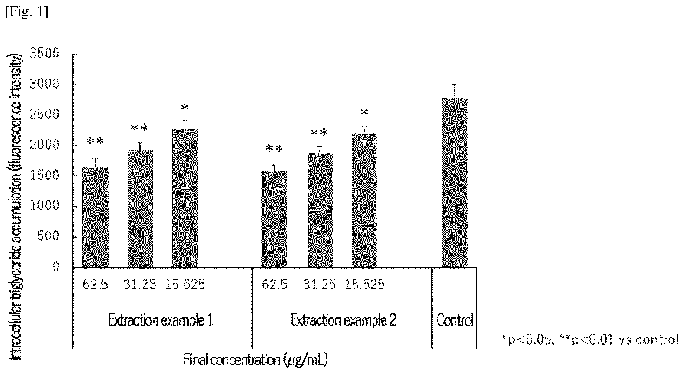

FIG. 1 is a graph showing the inhibitory effect on fat accumulation of a yerba mate crude extract and a decaffeinated yerba mate extract in tests using 3T3-L1 cells.

### [Description of embodiments]

The embodiments of the present invention will be explained in detail below. Note that the present invention can be implemented in many different embodiments and is not limited only to the specific forms described in the embodiments and examples shown below.

### Methods for production

The method for the production of the present invention (hereinafter referred to as "the method") is to remove caffeine from a yerba mate crude extract with an adsorbent to obtain a decaffeinated yerba mate extract.

The part of yerba mate (Ilex paraguariensis) used in the method is not limited and can be selected based on the purpose, and for example, the whole plant, aboveground parts, stems, leaves and roots can be used, with leaves being more preferred. Fresh, dried and processed leaves of yerba mate can be used.

The method of extracting yerba mate crude extract is not limited and can be carried out according to methods well known to those skilled in the art. Cold or warm water, alcoholic solvents, acetone or other organic solvents can be used as extraction solvents. Examples of alcoholic solvents include methanol, ethanol, propanol, isopropanol, butanol, isobutanol and mixtures thereof. Examples of organic solvents other than acetone include esters such as ethyl acetate; polyhydric alcohols such as ethylene glycol, propylene glycol and 1,3-butylene glycol; and ethers such as diethyl ether. These solvents may be used alone or in combination. The extraction solvent may be a combination of water and a hydrophilic organic solvent in the form of a hydrophilic solvent containing water. When using a combination of solvents, the mixing ratio of solvents can be set arbitrarily. Of these, water is more preferably used.

The amount of the extraction solvent is not limited as long as the composition exhibiting the above effects can be obtained, but it is preferably between 2 wt. part and 100 wt. part per 1 wt. part of dried yerba mate leaves. The extraction temperature is preferably between 4°C and 90°C. The extraction time is preferably between 30 minutes and one week. The extraction method can be any method such as agitation extraction, immersion extraction, countercurrent extraction, ultrasonic extraction and supercritical extraction.

A yerba mate extract from which caffeine has been removed (decaffeinated yerba mate extract) can be obtained by removing caffeine with an adsorbent from a yerba mate crude extract obtained through the above process, and then concentration and drying. The adsorbent is not limited as long as it is capable of removing caffeine and can be selected based on the purpose, and for example, acid white clay, synthetic adsorbent resins such as silica-magnesia adsorbents and styrenedivinylbenzene adsorbents can be used. Of these, acid white clay is more preferably used from the viewpoint of caffeine removal efficiency and retention of caffeoylquinic acids and dicaffeoylquinic acids contents. The use of acid white clay not only reduces caffeine by 90% or more but also maintains the caffeoylquinic acids and dicaffeoylquinic acids content at 10% or more by weight compared to without it.

The decaffeinated yerba mate extract may be the filtrate itself obtained by filtering the extract, a concentrated solution obtained by concentrating the filtrate, a dried product obtained by drying the concentrated solution, a crudely purified or finely purified product of any of these, or a mixture of any of these. Concentration can be carried out by any method, such as evaporative concentration, membrane concentration, etc. Drying can be carried out by any method, such as decompression drying, freeze-drying, spray-drying, etc. Excipients such as dextrin may be added if necessary. Purification can be carried out according to means known to those skilled in the art. For example, synthetic adsorbent resins, activated carbon, ion-exchange resins, gel filtration agents such as Sephadex and bio-gels, column chromatography, recrystallization, etc. may be used alone or in combination.

The composition can take the form of a food composition, a pharmaceutical composition or a cosmetic composition, and particularly in the case of a food composition, it can take the form of a food with functional claims or a health food. In the case of food, it can also take the form of a beverage as well as a solid product.

The form of the composition as a food composition, for example, can be a drink, a candy, a jelly, a gummy candy or other desserts, while the form as a health food or a food with functional claims, for example, can be a tablet, a hard capsule, a soft capsule, a granule, a drink, etc.

The form of the composition as a pharmaceutical composition can be a tablet, a capsule, a pill, a liquid, an emulsion, etc. The method of administration is not limited, but it is preferably in a form that can be administered orally. Various carriers can also be added to the extent that they are pharmaceutically acceptable. For example, the carriers can be an excipient, a coloring agent, a sweetening agent, a suspending agent, etc.

The form of the composition as a cosmetic composition can be, for example, a lotion, a cream, a pack, a gel, an emulsion, a balsam, an ointment, a liquid, a powder or a form that can be applied topically or internally.

The content of decaffeinated yerba mate (Ilex paraguariensis) extract in the composition can be adjusted according to the expected intake.

The daily intake of the decaffeinated yerba mate (Ilex paraguariensis) extract in the composition is not limited as long as it gives the effects of the composition but is preferably 10 mg or more and 1000 mg or less, more preferably 20 mg or more and 500 mg or less.

The decaffeinated yerba mate extract in the composition preferably contains 10 mg/g or less of caffeine therein, more preferably 5 mg/g or less. The total content of caffeoylquinic acids and dicaffeoylquinic acids in the yerba mate extract is preferably 10% or more by weight, more preferably 15% or more by weight. Inclusion in this range will preferably demonstrate the effects of the composition.

The caffeine content in yerba mate leaves has been reported to be from 1% to 2% by weight (see, for example, Non-patent document: Burris, K. P., Harte, F. M., Davidson, P. M., Stewart, Jr., C. N., & Zivanovic, S. (2012). Composition and bioactive properties of yerba mate (Ilex paraguariensis A. St.-Hil.): a review. Chilean Journal of Agricultural Research, 72(2), 268-274). When converted to the content in yerba mate extract, it ranges from 2.5% to 5.0% by weight.

The term "decaffeinated" refers to those with at least 90% of the caffeine removed. Reducing the caffeine content to one-tenth or less of that of normal yerba mate extract, which is calculated to be from 0.25% to 0.5% by weight, would decrease the potential risk of caffeine-induced side effects. In the present invention, the caffeine content is preferably 1.0% or less by weight, more preferably 0.5% or less by weight.

As described above, the present invention provides a composition that maintains its anti-obesity effect while removing caffeine.

### [Example]

Here, the compositions according to the above embodiments were actually produced, and their effects were tested. The following is a specific explanation.

### (1) Examination of adsorbent

One L of water was added to 100 g of dried yerba mate (Ilex paraguariensis) leaves and heated for 1 hour to obtain an extract after solid-liquid separation. The extract residue was heated again in 0.6 L of water for 1 hour to obtain an extract after solid-liquid separation. An adsorbent, either acid white clay or synthetic adsorbent, was added to the mixture of the first and second extracts to determine the adsorbent suitable for removing caffeine from a yerba mate crude extract. The result showed that acid white clay was most efficient in removing caffeine while maintaining caffeoylquinic acids content of 10% or more by weight (Table 2).

**[Table 2]**

| Adsorbent | Caffeine content | Caffeoylquinic acids content |
|---|---|---|
| Without adsorbent | 2.74% by weight | 12.04% by weight |
| Acid white clay | 0.19% by weight | 13.19% by weight |
| Synthetic adsorbent | 1.23% by weight | 8.76% by weight |

### (2) Extraction example 1 (production of decaffeinated yerba mate extract)

One L of water was added to 100 g of dried yerba mate (Ilex paraguariensis) leaves and heated for 1 hour to obtain an extract after solid-liquid separation. The extract residue was heated again in 0.6 L of water for 1 hour to obtain an extract after solid-liquid separation. The first and second extracts were mixed together, acid white clay was added, and then the mixture was stirred and filtered. The filtrate was concentrated under reduced pressure and dried to obtain 30 g of a solid.

The liquid chromatographic measurement of the solid obtained above demonstrated that the caffeine content in the decaffeinated yerba mate extract was 0.23% by weight. The total content of caffeoylquinic acids and dicaffeoylquinic acids was found to be 22.04% by weight.

### (3) Extraction example 2 (production of yerba mate crude extract)

One L of water was added to 100 g of dried yerba mate (Ilex paraguariensis) leaves and heated for 1 hour to obtain an extract after solid-liquid separation. The extract residue was heated again in 0.6 L of water for 1 hour to obtain an extract after solid-liquid separation. The first and second extracts were mixed together, and the filtrate was concentrated under reduced pressure and dried to obtain 30 g of a solid.

The liquid chromatographic measurement of the extract of extraction example 2 obtained above demonstrated that the caffeine content was 2.47% by weight. The total content of caffeoylquinic acids and dicaffeoylquinic acids was found to be 17.06% or more by weight.

### (4) Cell tests (intracellular triglyceride accumulation)

3T3-L1 cells were cultured in DMEM (high glucose) medium at 37°C and 5% CO₂. Samples were prepared to be 100,000 cells/mL, and insulin (final concentration: 5 µg/mL), dexamethasone (DEX, final concentration: 25 nM), and isobutylmethylxanthine (IBMX, final concentration: 0.5 mM) were used to induce differentiation into adipocytes. The extraction example 1 or 2 was added on the start date of differentiation induction to set their final concentrations at 62.5, 31.25 and 15.625 µg/mL. The medium was renewed every three days, and insulin (final concentration: 5 µg/mL), DEX (final concentration: 25 nM), IBMX (final concentration: 0.5 mM), the extraction example 1 or 2 (final concentrations: 62.5, 31.25 and 15.625 µg/mL) were newly added. DMSO was used as a control instead of the extraction example 1 or 2. After six days of differentiation induction, Nile red staining was used to determine the intracellular triglyceride accumulation (excitation wavelength: 525 nm, fluorescence wavelength: 585 nm).

The decaffeinated extract, extraction example 1, showed a concentration-dependent inhibitory effect on intracellular triglyceride accumulation, which was comparable to that of the extract of extraction example 2, the extract without decaffeination (FIG. 1). This indicates that the extract maintains its anti-obesity effect even when decaffeinated.

As described above, the cell tests have confirmed that the composition has an anti-obesity effect.

## Claims

1. A method for the production of a decaffeinated yerba mate (Ilex paraguariensis) extract, the method comprising a step of removing caffeine from a yerba mate (Ilex paraguariensis) crude extract with acid white clay.

2. A yerba mate extract prepared by removing caffeine from a yerba mate (Ilex paraguariensis) crude extract with acid white clay, the yerba mate extract comprising: 1.0% or less by weight of caffeine; and 10% or more by weight in total of caffeoylquinic acids and dicaffeoylquinic acids.

3. A yerba mate extract prepared by removing caffeine from a yerba mate (Ilex paraguariensis) crude extract with acid white clay, the yerba mate extract comprising: 0.5% or less by weight of caffeine; and 15% or more by weight in total of caffeoylquinic acids and dicaffeoylquinic acids.

4. An anti-obesity composition comprising as an active ingredient a yerba mate extract prepared by removing caffeine from a yerba mate (Ilex paraguariensis) crude extract with acid white clay, the yerba mate extract comprising: 1.0% or less by weight of caffeine; and 10% or more by weight in total of caffeoylquinic acids and dicaffeoylquinic acids.

5. An anti-obesity composition comprising as an active ingredient a yerba mate extract prepared by removing caffeine from a yerba mate (Ilex paraguariensis) crude extract with acid white clay, the yerba mate extract comprising: 0.5% or less by weight of caffeine; and 15% or more by weight in total of caffeoylquinic acids and dicaffeoylquinic acids.

6. An anti-obesity food composition, an anti-obesity pharmaceutical composition or an anti-obesity cosmetic composition comprising as an active ingredient a yerba mate extract according to either claim 2 or claim 3, the yerba mate extract prepared by removing caffeine from a yerba mate (Ilex paraguariensis) crude extract with acid white clay.

7. A decaffeination method comprising a step of removing caffeine from a yerba mate (Ilex paraguariensis) crude extract with acid white clay.

## Patentansprüche

1. Verfahren zur Herstellung eines entkoffeinierten Yerba-Mate-(Ilex-paraguariensis-)Extrakts, wobei das Verfahren einen Schritt des Entfernens von Koffein aus einem Yerba-Mate-(Ilex paraguariensis-)Rohextrakt mit Säure-Weißton umfasst.

2. Yerba-Mate-Extrakt, hergestellt durch Entfernen von Koffein aus einem Yerba-Mate-(Ilex-paraguariensis-)Rohextrakt mit Säure-Weißton, wobei der Yerba-Mate-Extrakt umfasst: 1,0 Gewichts-% oder weniger an Koffein; und insgesamt 10 Gewichts-% oder mehr an Caffeoylchinasäuren und Dicaffeoylchinasäuren.

3. Yerba-Mate-Extrakt, hergestellt durch Entfernen von Koffein aus einem Yerba-Mate-(Ilex-paraguariensis-)Rohextrakt mit Säure-Weißton, wobei der Yerba-Mate-Extrakt umfasst: 0,5 Gewichts-% oder weniger an Koffein; und insgesamt 15 Gewichts-% oder mehr an Caffeoylchinasäuren und Dicaffeoylchinasäuren.

4. Zusammensetzung gegen Adipositas, umfassend als Wirkstoff einen Yerba-Mate-Extrakt, hergestellt durch Entfernen von Koffein aus einem Yerba-Mate-(Ilex-paraguariensis-)Rohextrakt mit Säure-Weißton, wobei der Yerba-Mate-Extrakt umfasst: 1,0 Gewichts-% oder weniger an Koffein; und insgesamt 10 Gewichts-% oder mehr an Caffeoylchinasäuren und Dicaffeoylchinasäuren.

5. Zusammensetzung gegen Adipositas, umfassend als Wirkstoff einen Yerba-Mate-Extrakt, hergestellt durch Entfernen von Koffein aus einem Yerba-Mate-(Ilex-paraguariensis-)Rohextrakt mit Säure-Weißton, wobei der Yerba-Mate-Extrakt umfasst: 0,5 Gewichts-% oder weniger an Koffein; und insgesamt 15 Gewichts-% oder mehr an Caffeoylchinasäuren und Dicaffeoylchinasäuren.

6. Lebensmittelzusammensetzung gegen Adipositas, Arzneimittelzusammensetzung gegen Adipositas oder Kosmetikzusammensetzung gegen Adipositas, umfassend als Wirkstoff einen Yerba-Mate-Extrakt nach Anspruch 2 oder Anspruch 3, wobei der Yerba-Mate-Extrakt durch Entfernen von Koffein aus einem Yerba-Mate-(Ilex-paraguariensis-)Rohextrakt mit Säure-Weißton hergestellt ist.

7. Entkoffeinierungsverfahren, umfassend einen Schritt des Entfernens von Koffein aus einem Yerba-Mate-(Ilex-paraguariensis-)Rohextrakt mit Säure-Weißton.

## Revendications

1. Procédé de production d'un extrait décaféiné de yerba maté (Ilex paraguariensis), le procédé comprenant une étape d'élimination de caféine d'un extrait brut de yerba maté (Ilex paraguariensis) avec de l'argile blanche acide.

2. Extrait de yerba maté préparé par élimination de caféine d'un extrait brut de yerba maté (Ilex paraguariensis) avec de l'argile blanche acide, l'extrait de yerba maté comprenant : 1,0 % ou moins en poids de caféine ; et au total 10 % ou plus en poids d'acides caféoylquiniques et d'acides dicaféoylquiniques.

3. Extrait de yerba maté préparé par élimination de caféine d'un extrait brut de yerba maté (Ilex paraguariensis) avec de l'argile blanche acide, l'extrait de yerba maté comprenant : 0,5 % ou moins en poids de caféine ; et au total 15 % ou plus en poids d'acides caféoylquiniques et d'acides dicaféoylquiniques.

4. Composition anti-obésité comprenant en tant que principe actif un extrait de yerba maté préparé par élimination de caféine d'un extrait brut de yerba maté (Ilex paraguariensis) avec de l'argile blanche acide, l'extrait de yerba maté comprenant : 1,0 % ou moins en poids de caféine ; et au total 10 % ou plus en poids d'acides caféoylquiniques et d'acides dicaféoylquiniques.

5. Composition anti-obésité comprenant en tant que principe actif un extrait de yerba maté préparé par élimination de caféine d'un extrait brut de yerba maté (Ilex paraguariensis) avec de l'argile blanche acide, l'extrait de yerba maté comprenant : 0,5 % ou moins en poids de caféine ; et au total 15 % ou plus en poids d'acides caféoylquiniques et d'acides dicaféoylquiniques.

6. Composition alimentaire anti-obésité, composition pharmaceutique anti-obésité ou composition cosmétique anti-obésité comprenant en tant que principe actif un extrait de yerba maté selon la revendication 2 ou la revendication 3, l'extrait de yerba maté étant préparé par élimination de caféine d'un extrait brut deyerba maté (Ilex paraguariensis) avec de l'argile blanche acide.

7. Procédé de décaféination comprenant une étape d'élimination de la caféine d'un extrait brut de yerba maté (Ilex paraguariensis) avec de l'argile blanche acide.
